# FASCICULE DE BREVET EUROPEEN

(11) **EP 4 264 543 B1**
(45) Date de publication et mention de la délivrance du brevet: **28.01.2026**
(21) Numéro de dépôt: 21836120.2
(22) Date de dépôt: 10.12.2021
(51) Int. Cl.: G06T 7/10, G06T 7/00, G06T 7/62, A61B 5/00

(54) **PROCÉDÉ D'AIDE AU DIAGNOSTIC D'UNE MALADIE CARDIOVASCULAIRE D'UN VAISSEAU SANGUIN**
VERFAHREN ZUR UNTERSTÜTZUNG DER DIAGNOSE EINER HERZ-KREISLAUF-ERKRANKUNG EINES BLUTGEFÄSSES
METHOD FOR AIDING IN THE DIAGNOSIS OF A CARDIOVASCULAR DISEASE OF A BLOOD VESSEL

(30) Priorité: 15.12.2020 FR 2013257
(43) Date de publication de la demande: 25.10.2023
(60) Demande divisionnaire: 25226166.4
(73) Titulaire: Nurea, 33000 Bordeaux (FR)
(72) Inventeur: BERNARD, Florian, 33000 BORDEAUX (FR); LEGUAY, Romain, 33400 TALENCE (FR)
(74) Mandataire: Aquinov
(86) Numéro de dépôt international: PCT/EP2021/085268
(87) Numéro de publication internationale: WO 2022/128808

(56) Documents cités:
- US-A1- 2012 243 764
- US-A1- 2015 161 782
- US-A1- 2015 248 757
- US-A1- 2016 328 855
- US-A1- 2020 090 331
- ANDREAS GRÜNAUER: "Coronary Artery Tracking with Rule-based Gap Closing", 1 January 2011 (2011-01-01), XP055338815, Retrieved from the Internet <URL:https://publik.tuwien.ac.at/files/PubDat_201145.pdf> [retrieved on 20170125]
- CHUNLIANG WANG ET AL: "Integrating automatic and interactive methods for coronary artery segmentation: let the PACS workstation think ahead", INTERNATIONAL JOURNAL OF COMPUTER ASSISTED RADIOLOGY AND SURGERY, vol. 5, no. 3, 25 July 2009 (2009-07-25), DE, pages 275 - 285, XP055370566, ISSN: 1861-6410, DOI: 10.1007/s11548-009-0393-z

## Description

L'invention concerne le domaine du diagnostic de maladies ou de problèmes cardiovasculaires. Plus précisément, l'invention concerne un procédé d'aide au diagnostic d'une maladie cardiovasculaire d'un vaisseau sanguin, et notamment d'une aorte abdominale. L'art antérieur pertinent est connu par les documents US 2012/243764 A1, US 2015/161782 A1, US 2016/328855 A1, US 2020/090331 A1.

Un anévrisme d'une aorte abdominale (également appelé AAA) est une dilatation localisée, par exemple un gonflement ou une hypertrophie, de la paroi de l'aorte aboutissant à la formation d'une poche de taille variable, également appelé thrombus, autour du canal de l'aorte dans lequel circule le sang, également appelé lumière ou lumen. Cet anévrisme peut ainsi causer une restriction du diamètre interne de la lumière et/ou une augmentation du diamètre externe de l'aorte et crée ainsi un risque de compression des organes proches de l'aorte, un risque d'embolie ou encore un risque de rupture de l'aorte qui engendrerait une hémorragie interne.

Il est connu de détecter et de surveiller l'évolution d'un anévrisme à l'aide de procédés d'imagerie médicale, comme une angiographie par tomodensitométrie, également nommée CTA (de l'anglais «Computed Tomography Angiography »), ou par une IRM, imagerie par résonnance magnétique.

Dans le cas d'une CTA, un produit ou agent de contraste est injecté au patient pour améliorer la visibilité de l'aorte dans les angiographies. Le praticien obtient ainsi, à l'issue de la CTA, une pluralité d'angiographies représentant chacune une section de l'aorte. Afin de détecter un anévrisme, le praticien doit examiner toutes les angiographies pour détecter une variation locale importante du diamètre de l'aorte. Et il doit ensuite surveiller l'évolution de ce diamètre dans le temps. Cette méthode présente plusieurs inconvénients, et notamment ceux d'être une méthode manuelle, fastidieuse et longue et d'être une méthode praticiendépendante. En effet, l'étape de calcul du diamètre nécessite une sélection d'une image particulière et un repérage manuel sur cette image pour déterminer le diamètre de la lumière, de sorte qu'elle dépend du savoir-faire du praticien et n'est pas aisément reproductible d'une consultation à une autre.

Or, l'évolution du diamètre de l'anévrisme est l'un des paramètres essentiels dans le diagnostic de l'anévrisme et son traitement. En effet, la réparation d'un anévrisme est réalisée au travers d'une opération chirurgicale de type angioplastie, dans laquelle on ouvre l'anévrisme pour implanter une prothèse, également appelée stent, dans la lumière de l'aorte pour la dilater, ou encore par une procédure endovasculaire, dans lequel une endoprothèse est déployée à l'intérieur dans un vaisseau sanguin depuis une artère fémorale. Ces opérations étant risquées, la décision de procéder ou non à une telle opération est la résultante d'un compromis entre le risque de rupture et le risque de problème au cours de l'opération.

Il a été constaté que le taux de rupture d'un anévrisme augmente avec le diamètre de l'anévrisme. En d'autres termes, le risque de rupture s'estime en fonction du diamètre de la lumière de l'aorte. Il est à noter que d'autres indicateurs géométriques de l'aorte, comme son volume, permettent cette prise de décision. Il est ainsi nécessaire de pouvoir estimer ces indicateurs géométriques de façon simple, fiable, rapide, reproductible et non-praticien dépendante, en particulier de sorte que les mesures de ces indicateurs, par deux praticiens différents ou par un même praticien lors de deux consultations différentes, soient cohérentes et permettent une prise de décision fiable, ce qui n'est pas possible avec les méthodes existantes.

Par ailleurs, il est à noter que la surveillance de l'évolution de l'anévrisme ne s'arrête pas après la réparation de l'anévrisme. Il en effet nécessaire de vérifier que le diamètre de la lumière, malgré la pose d'un stent, est suffisant pour permettre la circulation du sang sans générer de nouveaux risques. En effet, la section de la lumière peut être finalement réduite du fait du stent, la circulation du sang générant dans ce cas des contraintes importantes sur les parois de l'aorte. Le même problème peut se poser lorsque l'aorte se calcifie, par exemple dans le cas d'une sténose aortique. Des dépôts calcaires apparaissent dans la lumière de l'aorte, contre les parois internes, ce qui génère un rétrécissement de la section circulante de l'aorte.

Il est ainsi nécessaire, lors de l'estimation du diamètre de l'aorte, ou d'un autre indicateur géométrique de l'aorte, de pouvoir détecter des éléments susceptibles de modifier le débit du sang dans l'aorte et qui viendraient ainsi impacter cet indicateur.

Il est à relever que des inconvénients équivalents sont constatés pour d'autres types de sténoses dans d'autres types de vaisseau sanguin.

La présente invention se place dans ce contexte et vise à répondre à ce besoin.

A ces fins, l'invention a pour objet un procédé d'aide au diagnostic d'une maladie cardiovasculaire d'un vaisseau sanguin, comprenant les étapes suivantes :
a. Fourniture d'une représentation tridimensionnelle d'un vaisseau sanguin d'un patient, obtenue par un dispositif d'imagerie médicale ;
b. Segmentation, à l'aide d'un classifieur, de ladite représentation tridimensionnelle pour obtenir une cartographie tridimensionnelle segmentée de ladite représentation tridimensionnelle, le classifieur étant agencé pour estimer si chaque voxel de la représentation tridimensionnelle appartient audit vaisseau sanguin et pour étiqueter ce voxel en fonction de cette estimation, ladite cartographie tridimensionnelle segmentée étant formée par l'ensemble des étiquettes attribuées par le classifieur aux voxels de la représentation tridimensionnelle ;
c. Comparaison de la valeur de chaque voxel d'une pluralité de voxels de la représentation tridimensionnelle, dont les étiquettes attribuées sur la cartographie tridimensionnelle sont celles du vaisseau sanguin, à une valeur seuil prédéterminée, une étiquette différente de celles du vaisseau sanguin étant attribuée à chaque voxel dont la valeur dépasse ladite valeur seuil prédéterminée ;
d. Détermination de l'évolution d'un indicateur géométrique du vaisseau sanguin le long de ce vaisseau sanguin au moyen des voxels de la représentation tridimensionnelle, dont les étiquettes attribuées sur la cartographie tridimensionnelle sont celles du vaisseau sanguin.

On comprend ainsi que grâce à l'invention, on procède d'une part à une segmentation automatique, au moyen du classifieur, de la représentation tridimensionnelle du vaisseau sanguin, de sorte à pouvoir sélectionner exclusivement les voxels de cette représentation qui correspondent effectivement au vaisseau sanguin, et notamment à la lumière du vaisseau sanguin. La sélection de ces voxels permet alors de procéder à un traitement de la représentation tridimensionnelle pour identifier, par seuillage, les voxels classifiés par erreur par le classifieur comme appartenant au vaisseau alors qu'ils correspondent à un stent agencé dans le vaisseau ou à une calcification du vaisseau. Il est alors possible de déterminer, de façon simple, rapide, fiable et reproductible, le diamètre réel de la lumière du vaisseau ou tout autre indicateur géographique .

Dans un exemple de réalisation de l'invention, la représentation tridimensionnelle fournie comporte un empilement d'angiographies tomodensitométriques du vaisseau sanguin du patient. Afin d'obtenir cet empilement, le patient est balayé, par exemple de façon hélicoïdale, par un faisceau de rayons X, de sorte à obtenir une pluralité d'images en coupe du vaisseau sanguin selon différentes incidences angulaires du faisceau irradiant. Chaque pixel de chaque image en coupe correspond donc à une unité de volume du patient, dont l'épaisseur correspond à la résolution de balayage. On comprend ainsi que l'assemblage de ces images permet de reconstituer numériquement un volume de points, en trois dimensions, appelé voxels, formant une représentation tridimensionnelle du vaisseau sanguin. Chaque voxel se voit attribué une valeur proportionnelle à l'absorption des rayons X par le tissu ou le matériau balayé correspondant. Cette valeur est mesurée en unité Hounsfield.

D'autres procédés de tomographie peuvent être employés dans le cadre de l'invention pour obtenir la pluralité d'angiographies tomodensitométriques, et notamment un procédé d'imagerie volumétrique par faisceau conique, selon lequel on procède à un unique balayage rotatif. On pourra également envisager d'employer d'autres techniques d'imagerie médicale permettant d'obtenir une représentation tridimensionnelle du vaisseau sanguin, comme de l'imagerie par résonnance magnétique.

Avantageusement, à l'issue de l'étape de segmentation, la cartographie tridimensionnelle est formée d'un ensemble de voxels, chaque voxel de la cartographie tridimensionnelle présentant les coordonnées d'un des voxels de la représentation tridimensionnelle et une intensité correspondant à l'étiquette attribuée par le classifieur à ce voxel de la représentation tridimensionnelle.

Dans un mode de réalisation de l'invention, le classifieur est agencé pour estimer, pour chaque voxel de la représentation tridimensionnelle, si :
a. ce voxel est en dehors du vaisseau sanguin, le classifieur attribuant dans ce cas une première étiquette à ce voxel,
b. ce voxel appartient à la lumière du vaisseau sanguin, le classifieur attribuant dans ce cas une deuxième étiquette à ce voxel,
c. ce voxel appartient à une tunique du vaisseau sanguin, le classifieur attribuant dans ce cas une troisième étiquette à ce voxel.

Par exemple, la première étiquette peut être une valeur nulle, la deuxième étiquette peut être une valeur de 1, et la troisième étiquette peut être une valeur de 2. On comprend ainsi que, dans cet exemple, les étiquettes du vaisseau sanguin sont les étiquettes non nulles. Ce mode de réalisation est particulièrement adapté pour une segmentation d'une représentation tridimensionnelle obtenue au moyen d'une angiographie par tomodensitométrie dans laquelle un produit ou agent de contraste est injecté au patient pour améliorer la visibilité du vaisseau sanguin dans les angiographies. Le produit de contraste permet en effet au classifieur de distinguer la lumière du vaisseau sanguin et les tissus formant les tuniques du vaisseau sanguin.

Il est également envisageable, dans un autre mode de réalisation de l'invention, d'opérer une segmentation d'une représentation tridimensionnelle obtenue au moyen d'une angiographie par tomodensitométrie sans produit de contraste, le classifieur attribuant dans ce cas seulement deux étiquettes, à savoir une première étiquette pour les voxels en dehors du vaisseau sanguin et une deuxième étiquette pour les voxels du vaisseau sanguin.

Avantageusement, l'étape de segmentation est mise en œuvre par un classifieur mettant en œuvre un algorithme d'apprentissage automatique, notamment du type réseau de neurones convolutifs.

La représentation tridimensionnelle du vaisseau sanguin est formée de « nuages de points » représentant chacune une partie bien définie du vaisseau sanguin. Il est ainsi possible de définir des frontières entre ces nuages, de sorte qu'il soit possible d'attribuer une étiquette aux voxels de ces parties. Ces frontières sont apprises, de façon automatique, en se basant sur un ensemble de représentations tridimensionnelles de référence aussi appelé ensemble d'apprentissage, les frontières de chaque représentation de cet ensemble d'apprentissage étant connues au préalable. Les règles permettant de décider de l'attribution ou non d'une étiquette à un voxel d'une nouvelle représentation tridimensionnelle, sont ainsi issues de l'apprentissage. On appelle ainsi classifieur mettant en œuvre un algorithme d'apprentissage automatique, un programme informatique dont le rôle est de décider quelle étiquette doit être attribué à un voxel d'une représentation tridimensionnelle fournie en entrée, en fonction des informations apprises. L'étiquette est déterminée par application des règles de décision (autrement appelées base de connaissances) qui ont elles-mêmes été préalablement apprises sur les données d'apprentissage.

Avantageusement, le procédé comporte une étape préalable d'apprentissage automatique supervisé du classifieur, mise en œuvre au moyen d'une pluralité de représentations tridimensionnelles prédéterminées. En d'autres termes, la pluralité de représentations tridimensionnelles prédéterminées forme un ensemble d'apprentissage pour le classifieur, lequel ajuste ainsi, de façon automatique, ses règles de décisions (et donc ses frontières), en fonction de l'étiquette qu'il attribue à chaque voxel de chaque représentation tridimensionnelle de l'ensemble d'apprentissage et l'étiquette réelle de ce voxel.

Si on le souhaite, le procédé peut comporter une étape préalable d'augmentation de l'ensemble d'apprentissage dans laquelle on génère de nouvelles représentations tridimensionnelles, à partir des représentations tridimensionnelles de l'ensemble d'apprentissage, qui sont distinctes de toutes les représentations tridimensionnelles de l'ensemble d'apprentissage. Par exemple, cette génération de nouvelles représentations tridimensionnelles pourra être réalisée en modifiant l'une des représentations tridimensionnelles de l'ensemble d'apprentissage de sorte à obtenir au moins une nouvelle représentation tridimensionnelle distincte de toutes les représentations tridimensionnelles de l'ensemble d'apprentissage. Cette modification pourra être réalisée notamment au moyen de l'une ou plusieurs des types de modifications suivants : dégradation de tout ou partie de la représentation tridimensionnelle initiale, changement de résolution, ajout d'un bruit, décalage dans une ou plusieurs dimensions, rotation.

Avantageusement, le classifieur est un réseau de neurones convolutifs, comprenant un chemin de contraction et un chemin d'expansion, dans lequel le chemin de contraction comprend une pluralité de couches de convolution associée chacune à une couche de correction agencée pour mettre en œuvre une fonction d'activation et de couches de sous-échantillonnage, chaque couche de sous-échantillonnage étant suivie d'au moins une couche de convolution, dans lequel le chemin d'expansion comprend une pluralité de couches de convolution et de couches de sur-échantillonnage, chaque couche de sur-échantillonnage étant suivie d'une couche de convolution. Les couches de sous-échantillonnage sont également appelées couches de « mise en commun » ou en anglais de « pooling ». Le cas échéant, la sortie de chaque couche de sur-échantillonnage peut être concaténée, avant d'entrer dans la couche de convolution suivante, à la carte d'activation issue d'une couche de convolution correspondante du chemin de contraction au travers d'un saut de connexion entre le chemin de contraction et le chemin d'expansion. Un tel réseau de neurones convolutifs est par exemple connu sous l'appellation « U-Net ».

Dans un mode de réalisation de l'invention, l'étape de segmentation comporte la segmentation au moyen du classifieur, de trois sections axiale, sagittale et coronale de ladite représentation tridimensionnelle pour obtenir trois cartographies bidimensionnelles segmentées et une étape de combinaison des cartographies bidimensionnelles pour obtenir ladite cartographie tridimensionnelle.

Selon cet exemple, ladite représentation tridimensionnelle peut être balayée selon trois axes vertical, horizontal et transversal pour obtenir une pluralité d'images de sections axiale, sagittale et coronale de la représentation tridimensionnelle, chaque image de section étant segmentée, au moyen du classifieur, de sorte à obtenir une cartographie bidimensionnelle segmentée de ladite image, le classifieur étant agencé pour estimer si chaque pixel de l'image appartient audit vaisseau sanguin et pour étiqueter ce pixel en fonction de cette estimation. Du fait du balayage, chaque étiquette associée à un pixel peut être repositionnée dans l'espace, de sorte à recombiner l'ensemble des cartographies bidimensionnelles pour former des voxels d'étiquettes, cet ensemble de voxels d'étiquettes formant alors la cartographie tridimensionnelle. Avantageusement, lorsque deux étiquettes, associées à des pixels d'images de sections obtenues selon deux axes distincts et qui correspondent à un même voxel, diffèrent, on attribue à ce voxel l'étiquette de valeur la plus haute.

Dans un mode de réalisation non limitatif de l'invention, le chemin de contraction peut recevoir en entrée une image de taille 256x256 pixels et comporter une pluralité, notamment quatre, blocs de contraction comportant chacun deux couches de convolution de type standard suivies d'une couche de sous-échantillonnage, la première couche de convolution du premier bloc de contraction recevant ladite image et la première couche de convolution des blocs de contraction suivants recevant en entrée la carte d'activation issue de la couche de sous-échantillonnage du bloc précédent . Si on le souhaite, chaque couche de convolution peut comporter une pluralité de noyaux de convolution (en anglais « Convolutional kernel ») de dimensions 3x3 et un pas de déplacement (en anglais « Stride ») de 1. Par exemple, le nombre de noyaux de convolution de chaque couche de convolution du premier bloc de contraction peut être de 64, et le nombre de noyaux de convolution de chaque couche de convolution des blocs de contraction suivants peut être le double du nombre de noyaux de convolution de chaque couche de convolution du bloc précédent. Si on le souhaite, chaque couche de correction associée à une couche de convolution peut être une couche d'unité linéaire rectifiée (en anglais « Rectified Unit Layer »). Si on le souhaite, chaque couche de sous-échantillonnage peut comporter un masque de sélection d'une valeur maximale (en anglais « Max pooling ») de dimensions 2x2 et un pas de déplacement de 2.

Avantageusement, le chemin de contraction et le chemin d'expansion peuvent être reliés l'un à l'autre par une pluralité, notamment deux, couches de convolution de type standard successives, comprenant chacune une pluralité de noyaux de convolution de dimensions 3x3 et un pas de déplacement de 1, le nombre de noyaux de convolution de chacune de ces couches de convolution étant le double du nombre de noyaux de convolution de chaque couche de convolution du dernier bloc de contraction.

Dans cet exemple, le chemin d'expansion peut recevoir en entrée la carte d'activation issue de la dernière couche de convolution et comporter une pluralité, notamment quatre, blocs d'expansion comportant chacun une couche de sur-échantillonnage suivie de deux couches de convolution de type standard, la couche de sur-échantillonnage du premier bloc d'expansion recevant ladite carte d'activation et la couche de sur-échantillonnage des blocs d'expansion suivants recevant en entrée la carte d'activation issue de la dernière couche de convolution du bloc précédent. Par exemple, chaque couche de sur-échantillonnage peut être agencée pour réaliser une opération de convolution transposée qui réalise un sur-échantillonnage et une interpolation depuis une pluralité de noyaux de convolution de dimensions 3x3 et de pas de déplacement de 2. Avantageusement toujours, le nombre de noyaux de convolution de la couche de sur-échantillonnage et de chaque couche de convolution du premier bloc d'expansion peut être identique au nombre de noyaux de convolution de chaque couche de convolution du dernier bloc de contraction, et le nombre de noyaux de convolution de la couche de sur-échantillonnage et de chaque couche de convolution des blocs d'expansion suivants peut être la moitié du nombre de noyaux de convolution de chaque couche de convolution du bloc précédent. Le cas échéant, la première couche de convolution d'un bloc d'expansion peut recevoir en entrée une concaténation de la carte d'activation issue de la couche de sur-échantillonnage de ce bloc d'expansion et de la carte d'activation, éventuellement rognée, issue de la dernière couche de convolution du bloc de contraction présentant le même nombre de noyaux de convolution.

Enfin, selon cet exemple, le classifieur peut comporter une dernière couche de convolution, apte à transformer les cartes d'activation issues du chemin d'expansion en un masque d'étiquette, en attribuant à chaque pixel de l'image de section qui est segmentée la classe présentant la plus haute probabilité. Par exemple, cette couche de convolution peut comprendre un noyau de convolution de dimensions 1x1, associée à une couche de correction de type exponentielle normalisée (en anglais « Softmax »).

Dans cet exemple, le classifieur peut par exemple être un réseau de neurones convolutifs de type « U-Net 2D » apte à segmenter des images, les hyperparamètres de ce classifieur, et notamment les poids des noyaux de convolution de toutes les couches de convolution et de sur-échantillonnage sont optimisés au cours de l'étape préalable d'apprentissage, notamment par une méthode de descente de gradient.

Dans un autre mode de réalisation de l'invention, lequel l'étape de segmentation peut être mise en œuvre directement sur la représentation tridimensionnelle pour obtenir ladite cartographie tridimensionnelle.

Le cas échéant, chaque couche de convolution peut comporter un noyau de convolution de dimensions 3x3x3 ou de dimensions 3x3 , et un pas de déplacement (en anglais « Stride ») de 1. Si on le souhaite, chaque couche de correction peut être une couche d'unité linéaire rectifiée (en anglais « Rectified Unit Layer »). Si on le souhaite, chaque couche de sous-échantillonnage peut comporter un masque de sélection d'une valeur maximale de dimensions 2x2x2 ou de dimensions 2x2 et un pas de déplacement de 2. Si on le souhaite, chaque couche de sur-échantillonnage peut être agencée pour réaliser une opération de convolution transposée qui réalise un sur-échantillonnage et une interpolation depuis un noyau de convolution de dimensions 3x3x3 ou de dimensions 2x2. Dans cet exemple, le classifieur peut par exemple être un réseau de neurones convolutifs de type « U-Net 3D » apte à segmenter une pile d'images.

Avantageusement, le procédé comprend, à l'issue de l'étape de segmentation et au préalable de l'étape de comparaison, une étape de confirmation et de correction des étiquettes attribuées par le classifieur aux voxels de la représentation tridimensionnelle. Cette étape de confirmation et de correction permet de corriger les faux positifs et faux négatifs introduits par le classifieur lors de l'étape de segmentation, de sorte à encore augmenter la fiabilité du procédé selon l'invention.

Selon un exemple de réalisation de l'invention, l'étape de confirmation et de correction comporte des opérations morphologiques réalisées sur la cartographie tridimensionnelle et notamment des opérations de type érosion et dilatation. Les opérations de type d'érosion permettent d'éliminer des aspects crénelés (également appelés en anglais « stair effect ») que pourraient présenter les contours de zones de la cartographie tridimensionnelle dont les voxels présentent une même étiquette à l'issue de la segmentation, ces aspects crénelés étant incohérents avec la morphologie d'un vaisseau sanguin. Les opérations de type dilatation permettent de regrouper des zones de la cartographie tridimensionnelle qui sont voisines mais distantes et dont les voxels présentent pourtant une même étiquette à l'issue de la segmentation, les tuniques et la lumière d'un vaisseau sanguin étant normalement continues.

Selon un exemple alternatif ou cumulatif de l'invention, l'étape de confirmation et de correction pourra comprendre une étape de propagation de l'étiquette des voxels d'une zone de la cartographie tridimensionnelle aux voxels d'une autre zone dont l'étiquette est différente, les zones formées par les voxels de la représentation tridimensionnelle correspondant à ces zones de la cartographie tridimensionnelle présentant une texture sensiblement identique. Le cas échéant, cette étape de propagation pourra comporter une étape de détermination de moyennes de gradients d'intensité et/ou d'intensité moyenne de voxels de la représentation tridimensionnelle afin de déterminer des zones de cette représentation tridimensionnelle dont les textures sont sensiblement identiques. Par exemple, deux zones pourront être considérées comme ayant des textures identique si les moyennes des gradients d'intensité de ces zones et/ou si les moyennes des intensités de ces zones différent, en valeur absolue, d'une valeur inférieure à un seuil fonction de l'écart-type de ces gradients d'intensité et/ou de ces intensités.

Par exemple, on pourra déterminer des moyennes de gradients d'intensité et/ou des intensités moyennes d'une première zone de voxels de la représentation tridimensionnelle auxquels a été attribuée la première étiquette et d'une deuxième zone de voxels de la représentation tridimensionnelle auxquels a été attribuée la deuxième étiquette, les voxels de ces première et deuxième zones étant situés de part et d'autre d'une frontière séparant la zone de première étiquette de la zone de deuxième étiquette dans la cartographie tridimensionnelle. Dans le cas où la première zone et la deuxième zone présentent une texture identique, on pourra propager la deuxième étiquette aux voxels de la première zone.

On pourra également, et notamment à la suite de la propagation précédente, déterminer des gradients d'intensité et/ou des intensités moyennes d'une deuxième zone de voxels de la représentation tridimensionnelle auxquels a été attribuée la deuxième étiquette et d'une troisième zone de voxels de la représentation tridimensionnelle auxquels a été attribuée la troisième étiquette, ces deuxième et troisième zones étant situées de part et d'autre d'une frontière séparant la zone de deuxième étiquette de la zone de troisième étiquette dans la cartographie tridimensionnelle. Dans le cas où la deuxième zone et la troisième zone présentent une texture identique, on pourra propager la troisième étiquette aux voxels de la deuxième zone.

Dans un mode de réalisation de l'invention, l'étape de comparaison comporte :
a. une première sous-étape de comparaison de la valeur de chaque voxel d'une pluralité de voxels de la représentation tridimensionnelle, dont les étiquettes attribuées sur la cartographie tridimensionnelle sont celles du vaisseau sanguin, à une première valeur seuil prédéterminée, une première étiquette associée à un stent étant attribuée à chaque voxel dont la valeur dépasse ladite première valeur seuil prédéterminée ;
b. une deuxième sous-étape de comparaison de la valeur de chaque voxel d'une pluralité de voxels de la représentation tridimensionnelle, dont les étiquettes attribuées sur la cartographie tridimensionnelle sont celles du vaisseau sanguin, à une deuxième valeur seuil prédéterminée inférieure à la première valeur seuil, une deuxième étiquette associée à une calcification étant attribuée à chaque voxel dont la valeur dépasse ladite deuxième valeur seuil prédéterminée.

Selon ces caractéristiques, il est ainsi possible de détecter dans un premier temps les voxels correspondant à un stent agencé dans le vaisseau sanguin, puis dans un deuxième temps, les voxels correspondant à une calcification du vaisseau sanguin, ces derniers présentant une intensité inférieure à celle des premiers. Par exemple, la première valeur seuil pourra être de 1500, et la deuxième valeur seuil pourra être de 500. Le cas échéant, l'étape de comparaison pourra comporter une étape de confirmation et de correction des première et deuxième étiquettes, correspondant respectivement à un stent et à une calcification, attribuées aux voxels de la représentation tridimensionnelle à l'issue des sous-étapes de comparaison, par exemple au moyen d'opérations morphologiques ou de propagation d'étiquette comme décrit précédemment. Grâce à ces caractéristiques, il est ainsi possible de déterminer l'évolution d'un indicateur géométrique traduisant le débit réel du sang dans le vaisseau sanguin, et pas seulement le débit théorique.

Avantageusement, l'étape de comparaison est mise en œuvre pour une pluralité de voxels dont les étiquettes attribuées sur la cartographie tridimensionnelle sont celles de la lumière du vaisseau sanguin et sont situées au niveau d'une frontière de délimitation de la cartographie tridimensionnelle entre les étiquettes de la lumière et les étiquettes des tuniques du vaisseau sanguin. On simplifie ainsi l'étape de comparaison, dans la mesure où un stent est destiné à venir contre les parois internes d'un vaisseau sanguin définissant sa lumière afin et qu'une calcification se forme normalement dans la lumière et contre ces parois internes.

Dans un mode de réalisation de l'invention, l'étape de détermination de l'évolution d'un indicateur géométrique du vaisseau sanguin est une étape de détermination de l'évolution du diamètre du vaisseau sanguin. Le cas échéant, cette étape de détermination comporte une étape d'estimation d'un graphe parcourant l'intégralité du vaisseau sanguin et dont chaque point est le barycentre des voxels situés dans une section de la représentation tridimensionnelle localement orthogonale au graphe et dont les étiquettes sont celles du vaisseau sanguin ; un diamètre local du vaisseau sanguin étant déterminé en fonction de chacun des points du graphe. On entend par graphe une succession de points où chaque point est connecté à au moins un autre point du graphe, de sorte qu'il soit possible de parcourir le vaisseau sanguin de bout en bout au moyen du graphe.

Par exemple, un premier point du graphe pourra être estimé en déterminant le barycentre des voxels situés dans la plus haute section de la représentation tridimensionnelle et dont les étiquettes sont celles du vaisseau sanguin. Le cas échéant, chaque point suivant du graphe pourra être déterminé en estimant le barycentre de l'intersection entre la représentation tridimensionnelle et une sphère, centrée sur le point précédent du graph et de rayon supérieur ou égal au plus petit rayon englobant les voxels situés dans la section de la représentation tridimensionnelle passant par ce point précédent du graph et dont les étiquettes sont celles du vaisseau sanguin, jusqu'à ce que l'intégralité de la représentation tridimensionnelle ait été parcourue. Par exemple, le rayon de la sphère pourra être égal audit plus petit rayon plus deux voxels. Cet algorithme présente l'avantage d'être particulièrement robuste vis-à-vis des ramifications que peut présenter un vaisseau sanguin. En effet, dans le cas d'une ramification du vaisseau sanguin, l'algorithme identifiera deux intersections entre la sphère et chaque branche du vaisseau sanguin, de sorte à pouvoir ensuite parcourir chacune de ces branches.

Si on le souhaite, ledit plus petit rayon pourra être celui englobant les voxels situés dans la section de la représentation tridimensionnelle passant par le point précédent du graph et dont les étiquettes sont celle de la lumière du vaisseau sanguin. En variante, ledit plus petit rayon pourra être celui englobant les voxels situés dans la section de la représentation tridimensionnelle passant par le point précédent du graph et dont les étiquettes sont celle de la lumière du vaisseau sanguin ou d'un stent ou d'une calcification.

Avantageusement, l'étape de détermination comporte en outre une étape d'estimation d'un nuage de points, chaque point du nuage de points étant le point localement le plus éloigné d'une frontière des voxels de la représentation tridimensionnelle du vaisseau sanguin et dont les étiquettes sont celles du vaisseau sanguin et une étape de correction des points du graphe à l'aide du nuage de points. Par exemple, chaque point pourra être déterminé en estimant les discontinuités du gradient de la fonction distance signée aux parois du vaisseau sanguin, notamment en estimant le barycentre des points de discontinuités de ce gradient, ces parois pouvant être matérialisées en déterminant une frontière de délimitation de la cartographie tridimensionnelle entre les première et deuxième étiquettes ou en déterminant une frontière de délimitation de la cartographie tridimensionnelle entre les deuxième et troisième étiquettes. Le cas échéant, l'étape de correction des points du graphe pourra être réalisée en minimisant la distance entre les points du nuage de points et les points du graphe.

Par exemple, pour chaque point du graphe, on pourra sélectionner le point du nuage du point situé à la plus petite distance de ce point du graph, par exemple au moyen d'un méthode des moindres carrés, ledit point du graph étant remplacé par ledit point du nuage de points sélectionné. Si on le souhaite, ledit remplacement pourra être conditionné par une contrainte de raideur du graphe. Par exemple, chaque branche du graphe pourra être représentée par un polynôme régulier, le remplacement d'un point de cette branche par un point du nuage de points sélectionné étant conditionné au fait que ce point sélectionné puisse être sensiblement représenté par ce polynôme.

L'invention a également pour objet un programme d'ordinateur comprenant un code de programme qui est conçu pour mettre en œuvre le procédé selon l'invention.

L'invention a également pour objet un support de données sur lequel est enregistré le programme d'ordinateur selon l'invention.

La présente invention est maintenant décrite à l'aide d'exemples uniquement illustratifs et nullement limitatifs de la portée de l'invention, et à partir des dessins annexés, dessins sur lesquels les différentes figures représentent :
[Fig. 1] représente, schématiquement et partiellement, un procédé d'aide au diagnostic d'une maladie cardiovasculaire d'un vaisseau sanguin selon un mode de réalisation de l'invention ;
[Fig. 2] représente, schématiquement et partiellement, un réseau de neurones de convolution employé dans le procédé de la [Fig. 1] ;
[Fig. 3] représente, schématiquement et partiellement, une étape du procédé de la [Fig. 1] ;
[Fig. 4] représente, schématiquement et partiellement, une autre étape du procédé de la [Fig. 1] ;
[Fig. 5] représente, schématiquement et partiellement, une autre étape du procédé de la [Fig. 1] ;
[Fig. 6] représente, schématiquement et partiellement, une autre étape du procédé de la [Fig. 1] ;
[Fig. 7] représente, schématiquement et partiellement, une autre étape du procédé de la [Fig. 1] ;
[Fig. 8] représente, schématiquement et partiellement, une autre étape du procédé de la [Fig. 1] ; et
[Fig. 9] représente, schématiquement et partiellement, une autre étape du procédé de la [Fig. 1].

Dans la description qui suit, les éléments identiques, par structure ou par fonction, apparaissant sur différentes figures conservent, sauf précision contraire, les mêmes références. En outre, les termes « avant », « arrière », « haut » et « bas », « sagittal », « axial » et « coronal » doivent être interprétés dans le contexte de l'orientation du vaisseau sanguin tel qu'il a été représenté, correspondant à l'orientation du vaisseau sanguin dans le corps humain.

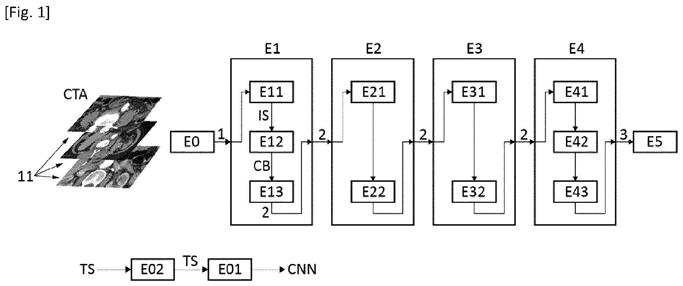

On a représenté en [Fig. 1] un procédé d'aide au diagnostic d'une maladie cardiovasculaire d'un vaisseau sanguin, en l'espèce d'un anévrisme A d'une aorte abdominale AA, d'un patient selon un exemple de réalisation de l'invention.

Au préalable du procédé, une représentation tridimensionnelle 1 de l'aorte AA du patient a été acquise par un procédé d'angiographie tomodensitométrique CTA. Dans ce procédé, le patient a été balayé, de façon hélicoïdale, par un faisceau de rayons X de sorte à obtenir une pluralité d'images en coupe 11 de l'aorte AA selon différentes incidences angulaires du faisceau irradiant. L'empilement de ces images 11, après un recalage en rotation, permet de reconstituer numériquement un volume de voxels, formant la représentation tridimensionnelle 1 de l'aorte AA, laquelle est fournie au procédé dans une première étape E0. Chaque voxel se voit attribué une valeur proportionnelle à l'absorption des rayons X par le tissu ou le matériau balayé correspondant. Cette valeur est mesurée en unité Hounsfield.

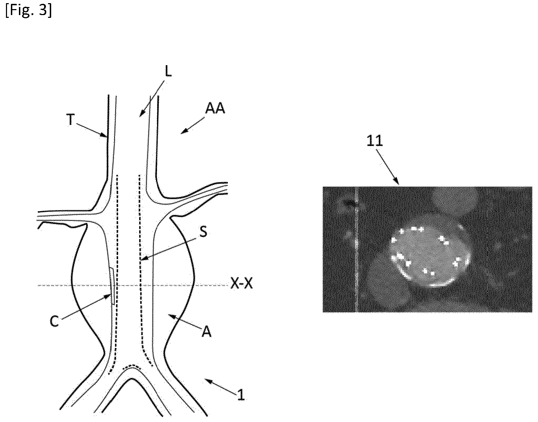

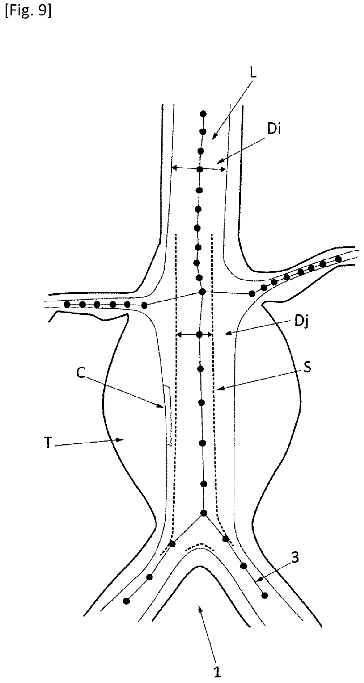

A des fins d'illustrations, on a représenté dans les [Fig. 3] à [Fig. 9] différentes vues, schématiques, de cette représentation tridimensionnelle 1. La [Fig. 3] représente ainsi, à gauche, une vue en coupe selon un plan coronal de la représentation tridimensionnelle 1 et, à droite, une angiographie 11 de la représentation tridimensionnelle 1 située au niveau d'un plan transversal X-X.

Dans l'exemple qui a été décrit, l'aorte AA présente un anévrisme A, entre la jonction de l'aorte AA et les artères rénales et la bifurcation de l'aorte AA vers les artères fémorales. L'aorte AA présente ainsi une lumière L dans laquelle le sang peut circuler et des tuniques T (intima, media, adventice) formant les parois de l'aorte AA autour de la lumière L. L'anévrisme A forme un thrombus autour de la lumière L. Par ailleurs, on constate que cet anévrisme A a été réparé au moyen d'un stent S posé dans la lumière L, par exemple au cours d'une angioplastie, et qu'une calcification C s'est formée sur la paroi interne de l'intima T. L'exemple qui a été décrit correspond ainsi à une consultation post-opératoire au cours de laquelle le praticien surveille l'évolution de l'anévrisme, étant entendu que le procédé pourrait être mis en œuvre lors d'une consultation de contrôle visant à détecter l'anévrisme ou à surveiller son évolution afin de décider si une angioplastie est judicieuse.

Lors de la CTA, un produit ou agent de contraste a été injecté au patient pour améliorer la visibilité de l'aorte dans les angiographies 11, et en particulier de distinguer sur chaque angiographie 11 les tuniques T et la lumière L. Toutefois, les frontières entre ces tuniques T et la lumière L ne sont pas représentées de façon claire sur ces angiographies, qui laissent en outre apparaitre d'autres tissus du corps du patient en dehors de l'aorte A.

Afin de pouvoir sélectionner uniquement les voxels de l'aorte A et de pouvoir clairement distinguer les frontières entre les tuniques T et la lumière L, le procédé comporte une étape E1 de segmentation de la représentation tridimensionnelle 1. Cette étape E1 est mise en œuvre au moyen d'un classifieur agencé pour estimer si chaque voxel de la représentation tridimensionnelle 1 appartient à l'aorte AA, et plus précisément à la lumière L ou aux tuniques T, et pour étiqueter ce voxel en fonction de cette estimation.

Dans l'exemple décrit, le classifieur met en œuvre un algorithme d'apprentissage automatique de type réseau de neurones convolutifs, également appelée CNN (de l'anglais « Convolutional Neural Network »).

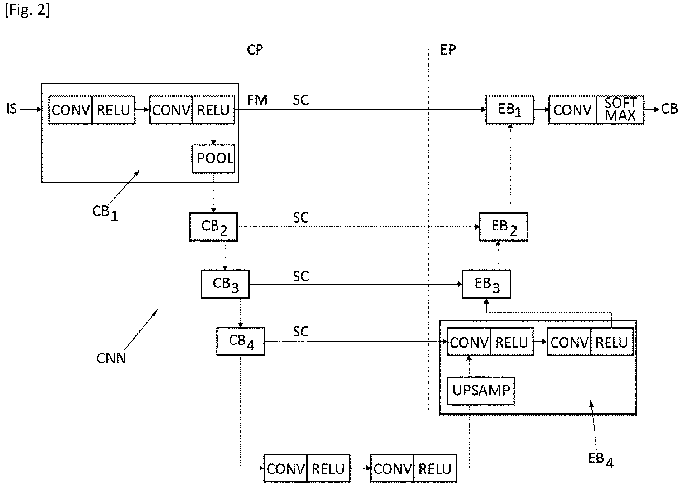

On a représenté en [Fig. 2] un exemple d'un classifieur CNN particulièrement adapté à la segmentation d'une représentation tridimensionnelle d'un vaisseau sanguin.

Le classifieur CNN de la [Fig. 2] comprend un chemin de contraction CP et un chemin d'expansion EP.

Le chemin de contraction CP comprend quatre blocs de contraction successifs CB₁ à CB₄. Chaque bloc de contraction comporte deux couches de convolution CONV, associée chacune à une couche de correction RELU agencée pour mettre en œuvre une fonction d'activation de type unité linéaire rectifiée, suivie par une couche de sous-échantillonnage ou « pooling » POOL. Chaque première couche de convolution CONV d'un bloc CBᵢ₊₁ reçoit ainsi la carte d'activation issue de la couche de sous-échantillonnage POOL du bloc précédent CBᵢ. Le nombre de noyaux de convolution des couches de convolution CONV d'un même bloc CBᵢ est identique, tandis que le nombre de noyaux de convolution des couches de convolution d'un bloc CBᵢ₊₁ est le double de celui du bloc CBᵢ. A noter que le nombre de noyaux de convolution des couches de convolution du bloc CB₁ est de 64. Ces noyaux de convolution sont de dimensions 3x3 et de pas de déplacement de 1. Chaque couche de sous-échantillonnage POOL comporte un masque de sélection d'une valeur maximale, de dimensions 2x2 et de pas de déplacement de 2.

Le chemin de contraction CP est relié au chemin d'expansion EP par deux couches de convolution CONV, comportant chacun le double de noyaux de convolution du bloc CB₄, ces noyaux étant de dimensions 3x3 et de pas de déplacement de 1.

Le chemin d'expansion EP comprend quatre blocs d'expansion successifs EB₄ à EB₁. Chaque bloc d'expansion comporte une couche de sur-échantillonnage UPSAMP suivie par deux couches de convolution CONV associée chacune à une couche de correction RELU. Chaque couche de sur-échantillonnage UPSAMP d'un bloc EBᵢ reçoit ainsi la carte d'activation issue de la dernière couche de convolution CONV du bloc EBᵢ₊₁ précédent. Le nombre de noyaux de convolution des couches de sur-échantillonnage UPSAMP et de convolution CONV d'un même bloc EBᵢ est identique, tandis que le nombre de noyaux de convolution des couches de sur-échantillonnage UPSAMP et de convolution CONV d'un bloc EBᵢ₊₁ est le double de celui du bloc EBᵢ. Ces noyaux de convolution sont de dimensions 3x3 et de pas de déplacement de 1 pour les couches de convolution CONV et de dimensions 3x3 et de pas de déplacement de 2 pour les couches UPSAMP. La sortie de chaque couche de sur-échantillonnage UPSAMP d'un bloc EBᵢ est concaténée, avant d'entrer dans la première couche de convolution de ce bloc EBᵢ, à la carte d'activation FM issue de la dernière couche de convolution CONV du bloc de contraction CBᵢ au travers de sauts de connexion SC entre le chemin de contraction CP et le chemin d'expansion EP.

Enfin, le classifieur CNN comprend une dernière couche de convolution CONV, recevant la carte d'activation issue de la dernière couche de convolution du bloc EB₁, et comportant un noyau de convolution de taille 1x1, associée à une couche de correction ou de normalisation SOFTMAX de type exponentielle normalisée.

Un tel réseau de neurones convolutifs est par exemple connu sous l'appellation « U-Net ».

Dans l'exemple décrit, ce classifieur CNN U-Net est un réseau dit « 2D », apte à segmenter des images. Dans une sous-étape E11 de l'étape E1, la représentation tridimensionnelle 1 est ainsi balayée selon trois axes horizontal X, vertical Y et transversal Z pour obtenir une pluralité d'images de sections IS respectivement sagittale, axiale et coronale de la représentation tridimensionnelle 1. Chacune de ces images IS est ainsi segmentée, dans une étape E12, au moyen du classifieur U-Net 2D, pour estimer si chaque pixel de l'image IS appartient à l'aorte AA et pour étiqueter ce pixel en fonction de cette estimation

Plus précisément, le classifieur CNN est agencé pour estimer, pour chaque pixel d'une image IS qu'il doit segmenter, si :
a. ce pixel est en dehors de l'aorte AA, le classifieur CNN attribuant dans ce cas une première étiquette à ce pixel, par exemple une étiquette de valeur 0,
b. ce pixel appartient à la lumière L de l'aorte AA, le classifieur CNN attribuant dans ce cas une deuxième étiquette à ce pixel, par exemple une étiquette de valeur 1 ;
c. ce pixel appartient à une T de l'aorte AA, le classifieur CNN attribuant dans ce cas une troisième étiquette à ce pixel, par exemple une étiquette de valeur 2.

La dernière couche de convolution CONV associée à la couche de correction SOFTMAX du classifieur CNN permet de transformer les cartes d'activation FM issues du chemin d'expansion EP en un masque d'étiquettes CB, en attribuant à chaque pixel de l'image IS devant être segmentée l'étiquette présentant la plus haute probabilité. Le masque d'étiquettes CB présente ainsi des dimensions identiques à celles de l'image IS devant être segmentée et forme ainsi une cartographie bidimensionnelle de cette image IS.

Afin de pouvoir segmenter de façon correcte une nouvelle image IS, le classifieur CNN a subi une étape préalable d'apprentissage automatique E01, dit supervisé. Dans cette étape, le classifieur CNN a successivement segmenté une pluralité d'images de section respectivement sagittale, axiale et coronale d'une pluralité de représentations tridimensionnelles prédéterminées dont les étiquettes des voxels sont connues d'avance. Cette pluralité de représentations tridimensionnelles prédéterminées forme un ensemble d'apprentissage TS pour le classifieur CNN. Le CNN peut ainsi déterminer pour chaque étiquette qu'il attribue à un pixel d'une image issue de cet ensemble d'apprentissage TS s'il a commis une erreur et peut ajuster, de façon automatique en fonction de cette erreur, ses hyperparamètres, à savoir les poids des noyaux de convolution des couches de convolutions CONV et des couches de sur-échantillonnage UPSAMP. Cet ajustement peut par exemple être mis en œuvre par une méthode de descente de gradient.

Dans l'exemple décrit, l'ensemble d'apprentissage TS a été artificiellement augmenté, dans une étape préalable E02. Dans cette étape E02, on a généré de nouvelles représentations tridimensionnelles, en modifiant les représentations tridimensionnelles de l'ensemble d'apprentissage TS de sorte à obtenir au moins des nouvelles représentations tridimensionnelles distinctes de toutes les représentations tridimensionnelles de l'ensemble d'apprentissage TS, par exemple par des opérations de dégradation, de changement de résolution, d'ajout d'un bruit, de décalage dans une ou plusieurs dimensions et/ou de rotation. Ces nouvelles représentations tridimensionnelles ont été ajoutées à l'ensemble d'apprentissage TS. Il a ainsi été possible, partant d'un jeu de données réelles relativement limité, d'obtenir un ensemble d'apprentissage TS particulièrement conséquent, afin de pouvoir entrainer le classifieur CNN de façon optimale.

Dans une sous-étape E13 de l'étape E1, à l'issue de l'étape E12, les cartographies bidimensionnelles CB obtenues par la segmentation des images de section IS respectivement sagittale, axiale et coronale de la représentation tridimensionnelle 1 ont été combinées pour former une cartographie tridimensionnelle 2 de cette représentation tridimensionnelle 1.

En effet, les pixels des images de section IS peuvent être positionnés dans l'espace, les coordonnées des images de section IS étant connues du fait du balayage. Dès lors, chaque étiquette associée à un pixel peut être repositionnée dans l'espace, de sorte à recombiner l'ensemble des cartographies bidimensionnelles CB pour former des voxels d'étiquettes, cet ensemble de voxels d'étiquettes formant alors la cartographie tridimensionnelle 2.

Dans l'exemple décrit, lorsque deux étiquettes, associées à des pixels d'images de section IS obtenues selon deux axes distincts et qui correspondent à un même voxel, présentent des valeurs distinctes, on attribue à ce voxel l'étiquette de valeur la plus haute parmi ces deux étiquettes.

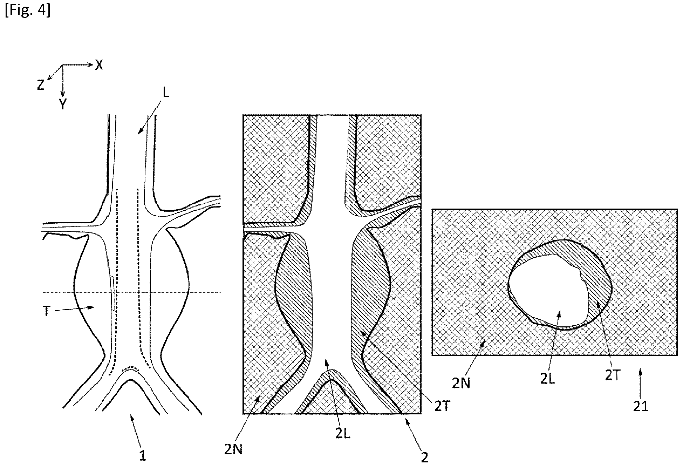

On a ainsi représenté en [Fig. 4], à gauche, la vue en coupe selon un plan coronal de la représentation tridimensionnelle 1, comme montré en [Fig. 3] ; au centre, une vue en coupe selon le même plan coronal de la cartographie tridimensionnelle 2 ; et, à droite, une section 21 de la cartographie tridimensionnelle 2 située au niveau d'un plan transversal X-X.

On constate ainsi que la représentation tridimensionnelle 1 a bien été segmentée, au niveau de la cartographie tridimensionnelle 2, en trois volumes de points 2N, 2L et 2T, correspondant respectivement aux étiquettes de valeurs 0, 1 et 2. Toutefois, la segmentation opérée par le CNN est une méthode statistique, qui peut introduire des erreurs.

Afin de détecter, et le cas échéant, de corriger ces erreurs, le procédé comprend une étape E2 de confirmation et de correction des étiquettes attribuées par le classifieur CNN aux voxels de la représentation tridimensionnelle 1.

Cette étape E2 consiste, dans l'exemple décrit, d'une part, à réaliser des opérations morphologiques de type érosion et dilatation sur la cartographie tridimensionnelle 2, et, d'autre part, à propager l'étiquette des voxels d'une zone de la cartographie tridimensionnelle 2 aux voxels d'une autre zone dont l'étiquette est différente mais dont la texture est sensiblement identique.

Dans l'exemple décrit, le procédé comporte ainsi une sous-étape E21 de détermination de moyennes de gradients d'intensité et/ou des intensités moyennes de premières zones Z1 de voxels de la représentation tridimensionnelle 1 auxquels a été attribuée la première étiquette de valeur 0 et de deuxièmes zones Z2 de voxels de la représentation tridimensionnelle 1 auxquels a été attribuée la deuxième étiquette de valeur 1, ces zones Z1 et Z2 étant situés de part et d'autre d'une frontière séparant le volume de points 2T et le volume de points 2N dans la cartographie tridimensionnelle 2. Dans le cas où une première zone Z1 et une deuxième zone Z2 voisines présentent une même intensité moyenne et/ou une même moyenne de gradient d'intensité d'une zone à l'autre, la deuxième étiquette de valeur 1 sera attribuées aux voxels de la première zone Z1. Dans cet exemple, deux moyennes de gradients ou d'intensités sont considérées identiques si la valeur absolue de leur différence est inférieure à un seuil proportionnel à l'écart-type de ces gradients et de ces intensités.

Le procédé comporte également, à la suite de l'étape E21, une sous-étape E22 de déterminer des moyennes de gradients d'intensité et/ou des intensités moyennes de deuxièmes zones Z2 de voxels de la représentation tridimensionnelle 1 auxquels a été attribuée la deuxième étiquette de valeur 1 et de troisièmes zones Z3 de voxels de la représentation tridimensionnelle 1 auxquels a été attribuée la troisième étiquette de valeur 2, ces zones Z2 et Z3 étant situés de part et d'autre d'une frontière séparant le volume de points 2T et le volume de points 2L dans la cartographie tridimensionnelle 2. Dans le cas où une deuxième zone Z2 et une troisième zone Z3 voisines présentent une même intensité moyenne et/ou une même moyenne de gradient d'intensité d'une zone à l'autre, la troisième étiquette de valeur 2 sera attribuées aux voxels de la deuxième zone Z2.

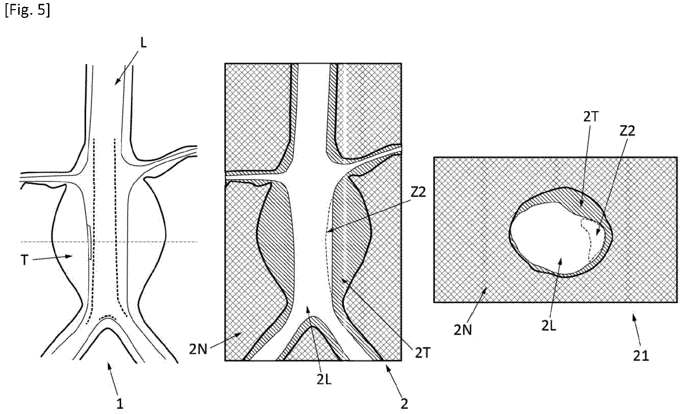

On a ainsi représenté en [Fig. 5], à gauche, la vue en coupe selon un plan coronal de la représentation tridimensionnelle 1, au centre, la vue en coupe selon le même plan coronal de la cartographie tridimensionnelle 2 à l'issue de l'étape E2 ; et, à droite, une section 21 de la cartographie tridimensionnelle 2 située au niveau d'un plan transversal X-X.

On constate ainsi qu'une deuxième zone Z2, auparavant étiquetée par le classifieur CNN comme faisant partie de la tunique, a été corrigée, son étiquette étant désormais la troisième étiquette de valeur 2, indiquant ainsi que les voxels de cette zone Z2 dans la représentation tridimensionnelle 1 font en réalité partie de la lumière L. Bien que les volumes de points 2N, 2L et 2T soient désormais fiables, certains voxels de la représentation tridimensionnelle 1 nécessitent un étiquetage particulier, afin d'identifier le stent S et la calcification C.

A ces fins, le procédé comporte une étape E3 de comparaison de la valeur de chaque voxel d'une pluralité de voxels de la représentation tridimensionnelle 1, dont les étiquettes attribuées sur la cartographie tridimensionnelle 2 sont celles de l'aorte, à savoir les étiquettes de valeurs 1 et 2, à une valeur seuil prédéterminée, une étiquette différente de celles du vaisseau sanguin étant attribuée à chaque voxel dont la valeur dépasse ladite valeur seuil prédéterminée.

Plus précisément, dans l'exemple décrit, l'étape de comparaison E3 comporte une première sous-étape E31 de comparaison de la valeur de chaque voxel dont l'étiquette attribuée sur la cartographie tridimensionnelle 2 est la troisième étiquette de valeur 2 et qui est situé au niveau d'une frontière séparant le volume de points 2T et le volume de points 2L dans la cartographie tridimensionnelle 2, à une première valeur seuil prédéterminée. Une étiquette, par exemple de valeur 3, associée à un stent, est attribuée à chaque voxel dont la valeur dépasse ladite première valeur seuil prédéterminée.

L'étape de comparaison E3 comporte une également une deuxième sous-étape E32 de comparaison de la valeur de chaque voxel dont l'étiquette attribuée sur la cartographie tridimensionnelle 2 est la troisième étiquette de valeur 2 et qui est situé au niveau d'une frontière séparant le volume de points 2T et le volume de points 2L dans la cartographie tridimensionnelle 2, à une deuxième valeur seuil prédéterminée inférieure à la première valeur seuil. Une étiquette, par exemple de valeur 4, associée à une calcification, est attribuée à chaque voxel dont la valeur dépasse ladite deuxième valeur seuil prédéterminée.

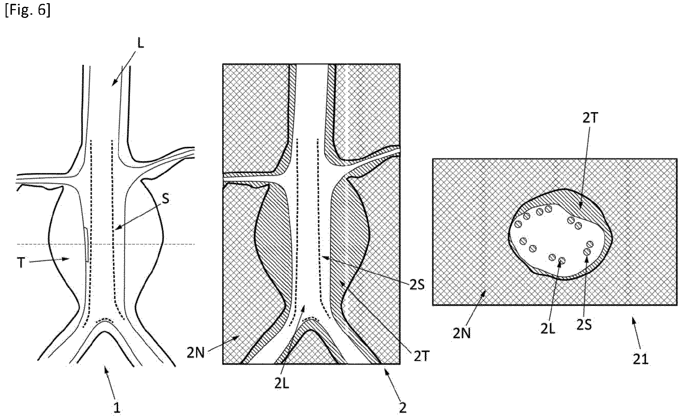

On a ainsi représenté en [Fig. 6], à gauche, la vue en coupe selon un plan coronal de la représentation tridimensionnelle 1, au centre, la vue en coupe selon le même plan coronal de la cartographie tridimensionnelle 2 à l'issue de l'étape E31 ; et, à droite, une section 21 de la cartographie tridimensionnelle 2 située au niveau d'un plan transversal X-X.

On constate l'apparition, dans la cartographie tridimensionnelle, de voxels 2S, dans le volume de points 2L, au voisinage de la frontière de ce volume de points 2L avec le volume de points 2T, qui présentent une étiquette distincte de celle de la lumière L. Il s'agit ainsi de voxels 2S correspondant au stent S.

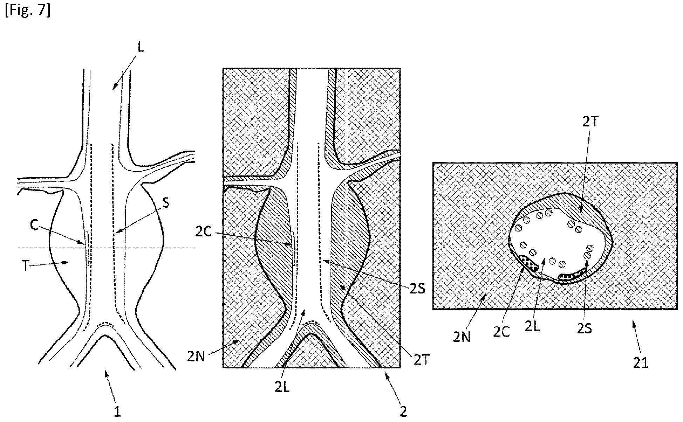

On a également représenté en [Fig. 7], à gauche, la vue en coupe selon un plan coronal de la représentation tridimensionnelle 1, au centre, la vue en coupe selon le même plan coronal de la cartographie tridimensionnelle 2 à l'issue de l'étape E32 ; et, à droite, une section 21 de la cartographie tridimensionnelle 2 située au niveau d'un plan transversal X-X.

On constate l'apparition, dans la cartographie tridimensionnelle, de voxels 2C, dans le volume de points 2L, au voisinage de la frontière de ce volume de points 2L avec le volume de points 2T, qui présentent une étiquette distincte de celle de la lumière L. Il s'agit ainsi de voxels 2C correspondant à la calcification C.

A l'issue de l'étape 3, on dispose ainsi d'une cartographie tridimensionnelle 2 de l'aorte AA identifiant de façon fiable tous voxels de la représentation tridimensionnelle 1 appartenant à un même élément de l'aorte.

Le procédé comporte une étape E4 de détermination de l'évolution d'un indicateur géométrique de l'aorte le long de ce vaisseau sanguin, au moyen des voxels de la représentation tridimensionnelle 1, dont les étiquettes attribuées sur la cartographie tridimensionnelle 2 sont celles du vaisseau sanguin.

Dans l'exemple décrit, cette étape E4 est une étape de détermination de l'évolution du diamètre réel de la lumière L de l'aorte, c'est-à-dire celui permettant effectivement la circulation du sang.

Dans une sous-étape E41, on estime un graphe 3 parcourant l'intégralité de l'aorte AA, et dont chaque point 3i est le barycentre des voxels situés dans une section de la représentation tridimensionnelle 1 localement orthogonale au graphe et dont les étiquettes sont celles de la lumière L.

A ces fins, le premier point du graph 31 correspond au barycentre des voxels situés dans la plus haute section coronale de la représentation tridimensionnelle 1 et dont les étiquettes sont celles de la lumière L de l'aorte AA.

Une sphère S31 est positionnée sur ce premier point 31, le rayon de cette sphère S31 étant tel que la sphère S31 englobe tous les voxels situés dans la section coronale de la représentation tridimensionnelle 1 passant par le point 31 et dont les étiquettes sont celles de la lumière L.

Cette sphère S31 intersecte la lumière L en une intersection C31, dont on détermine alors le barycentre, lequel forme le deuxième point 32 du graph 3.

Chaque point suivant 3j du graphe peut ainsi être déterminé en estimant le barycentre de l'intersection entre la lumière L et une sphère S3i, centrée sur le point précédent 3i du graph 3 et de rayon supérieur ou égal au plus petit rayon englobant les voxels situés dans la section C3i de la lumière L passant par le point précédent 3i du graph 3 et dont les étiquettes sont celle de la lumière L, jusqu'à ce que l'intégralité de la représentation tridimensionnelle 1 ait été parcourue.

On constate en particulier que cette étape E41 permet, lorsque l'aorte AA présente une ramification, d'identifier une intersection C3i entre la sphère S3i et chaque branche de l'aorte. On peut alors dupliquer l'algorithme pour parcourir chacune de ces branches, à partir du barycentre 3j de chacune de ces intersections.

L'ensemble des points 3i forme ainsi un graphe 3 où chaque point est connecté à au moins un autre point du graphe, de sorte qu'il soit possible de parcourir l'aorte AA de bout en bout au moyen du graphe 3.

Dans une sous étape E42, on estime détermine également le gradient de la fonction distance signée aux parois de la lumière L de l'aorte AA, c'est-à-dire la frontière séparant le volume de points 2L du volume de points 2T. Les barycentres des points de discontinuités de ce gradient, par exemple déterminés dans chaque coupe orthogonale à l'aorte AA selon le graphe 3, forment ainsi un nuage de points 4, chaque point 4i du nuage de points 4 étant le point localement le plus éloigné de cette frontière.

Dans une étape E43, on détermine, pour chaque point 3i du graphe 3, le point 4i du nuage de points 4 le plus proche de ce point 3i, par une méthode des moindres carrés. Les coordonnées spatiales du point 3i du graphe 3 sont alors remplacées par celles du point 4i. Il est à noter que ce remplacement est conditionné au fait que les coordonnées spatiales du point 4i respectent sensiblement une équation représentant la branche du graphe 3 sur laquelle est positionné le point 3i.

Le graphe 3, à l'issue de l'étape E43, permet ainsi de parcourir l'ensemble de l'aorte AA tout en représentant les points de la lumière L les plus éloignés des parois de cette lumière L.

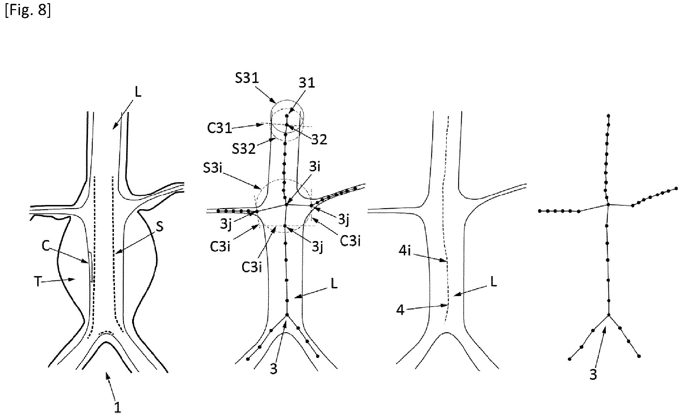

On a représenté en [Fig. 8], successivement de gauche à droite :
a. la vue en coupe selon un plan coronal de la représentation tridimensionnelle 1 ;
b. une vue en coupe de la lumière L selon le même plan coronal de la cartographie tridimensionnelle 2, incluant le graphe 3 déterminé à l'issue de l'étape E41 ;
c. une vue en coupe de la lumière L selon le même plan coronal de la cartographie tridimensionnelle 2, incluant le nuage de points 4 déterminé à l'issue de l'étape E42 ;
d. une vue en coupe du graphe 3 selon le même plan coronal de la cartographie tridimensionnelle 2, déterminé à l'issue de l'étape E43.

Chacun des points 3i du graphe 3 permet ainsi de déterminer, dans une étape E5, le diamètre local Di de la lumière L, dans une section de cette lumière L localement orthogonale au graphe 3 passant par ce point 3i, ce diamètre Di étant le diamètre de la lumière L entre ses parois si cette section est dépourvue de voxels dont les étiquettes sont celles du stent S ou de la calcification C, ou, dans le cas contraire, un diamètre de la lumière L prenant en compte ces voxels dont les étiquettes sont celles du stent S ou de la calcification C.

On a ainsi représenté en [Fig. 9] la vue en coupe selon un plan coronal de la représentation tridimensionnelle 1, comme représentée en [Fig. 1], à laquelle ont été ajoutés le graphe 3 et deux diamètres locaux Di et Dj, déterminés à l'issue de l'étape E5.

La description qui précède explique clairement comment l'invention permet d'atteindre les objectifs qu'elle s'est fixée, à savoir, pouvoir estimer un indicateur géométrique réel d'un vaisseau sanguin de façon simple, fiable, rapide, reproductible et non-praticien dépendante, en proposant un procédé dans lequel une représentation tridimensionnelle du vaisseau sanguin est segmentée automatique, au moyen du classifieur, de sorte à pouvoir sélectionner exclusivement les voxels de cette représentation qui correspondent effectivement au vaisseau sanguin, puis dans lequel la représentation tridimensionnelle est traitée pour identifier, par seuillage, les voxels classifiés par erreur par le classifieur comme appartenant au vaisseau alors qu'ils correspondent à un stent agencé dans le vaisseau ou à une calcification du vaisseau.

En tout état de cause, l'invention ne saurait se limiter aux modes de réalisation spécifiquement décrits dans ce document, et s'étend en particulier à tous moyens équivalents et à toute combinaison techniquement opérante de ces moyens. On pourra en particulier envisager d'employer le procédé sur d'autres types de représentation tridimensionnelle d'un vaisseau sanguin, comme par exemple celles issues d'autres techniques d'imagerie médicale, comme de l'imagerie par résonnance magnétique, ou encore comme celles issues d'une angiographie par tomodensitométrie sans produit de contraste.

On pourra également envisager d'autres types de classifieur permettant de segmenter la représentation tridimensionnelle, comme par exemple un classifieur de type « U-Net 3D » apte à segmenter directement la représentation tridimensionnelle pour obtenir ladite cartographie tridimensionnelle, en utilisant des noyaux de convolution tridimensionnels. On pourra également envisager d'utiliser d'autres types de réseaux de neurones de convolution, voire d'autres types de classifieur mettant en œuvre un algorithme d'apprentissage automatique.

On pourra également envisager de déterminer, alternativement ou cumulativement, l'évolution d'autres indicateurs géométriques du vaisseau sanguin que son diamètre, et notamment son volume.

## Revendications

1. Procédé d'aide au diagnostic d'une maladie cardiovasculaire (A)d'un vaisseau sanguin (AA), mise en œuvre par ordinateur et comprenant les étapes suivantes :
a. (E0) Fourniture d'une représentation tridimensionnelle (1) d'un vaisseau sanguin (AA) d'un patient, obtenue par un dispositif d'imagerie médicale ;
b. (E1) Segmentation, à l'aide d'un classifieur (CNN), de ladite représentation tridimensionnelle pour obtenir une cartographie tridimensionnelle segmentée (2) de ladite représentation tridimensionnelle, le classifieur étant agencé pour estimer si chaque voxel de la représentation tridimensionnelle appartient audit vaisseau sanguin et pour étiqueter ce voxel en fonction de cette estimation, ladite cartographie tridimensionnelle segmentée étant formée par l'ensemble des étiquettes attribuées par le classifieur aux voxels de la représentation tridimensionnelle ;
c. (E3) Comparaison de la valeur de chaque voxel d'une pluralité de voxels de la représentation tridimensionnelle, dont les étiquettes attribuées sur la cartographie tridimensionnelle sont celles du vaisseau sanguin, à une valeur seuil prédéterminée, une étiquette différente de celles du vaisseau sanguin étant attribuée à chaque voxel dont la valeur dépasse ladite valeur seuil prédéterminée;
d. (E4) Détermination de l'évolution d'un indicateur géométrique (Di, Dj) du vaisseau sanguin le long de ce vaisseau sanguin au moyen des voxels de la représentation tridimensionnelle, dont les étiquettes attribuées sur la cartographie tridimensionnelle sont celles du vaisseau sanguin ;
dans lequel le classifieur (CNN) est agencé pour estimer, pour chaque voxel de la représentation tridimensionnelle (1), si :
i. ce voxel est en dehors du vaisseau sanguin (AA), le classifieur attribuant dans ce cas une première étiquette à ce voxel,
ii. ce voxel appartient à la lumière (L) du vaisseau sanguin, le classifieur attribuant dans ce cas une deuxième étiquette à ce voxel,
iii. ce voxel appartient à une tunique (T) du vaisseau sanguin, le classifieur attribuant dans ce cas une troisième étiquette à ce voxel ;
dans lequel l'étape de segmentation (E1) est mise en œuvre par un classifieur (CNN) mettant en œuvre un algorithme d'apprentissage automatique
et dans lequel l'étape de segmentation (E1) comporte la segmentation (E12) au moyen du classifieur (CNN), de trois sections (IS) axiale, sagittale et coronale de ladite représentation tridimensionnelle (1) pour obtenir trois cartographies bidimensionnelles segmentées (CB) et une étape de combinaison (E13) des cartographies bidimensionnelles pour obtenir ladite cartographie tridimensionnelle (2).

2. Procédé selon la revendication précédente, dans lequel le classifieur (CNN) est un réseau de neurones convolutifs, comprenant un chemin de contraction (CP) et un chemin d'expansion (EP), dans lequel le chemin de contraction comprend une pluralité de couches de convolution (CONV) associée chacune à une couche de correction (RELU) agencée pour mettre en œuvre une fonction d'activation et de couches de sous-échantillonnage (POOL), chaque couche de sous-échantillonnage étant suivie d'au moins une couche de convolution, dans lequel le chemin d'expansion comprend une pluralité de couches de convolution (CONV) et de couches de sur-échantillonnage (UPSAMP), chaque couche de sur-échantillonnage étant suivie d'une couche de convolution.

3. Procédé selon la revendication précédente, dans lequel la sortie de chaque couche de sur-échantillonnage (UPSAMP) est concaténée, avant d'entrer dans la couche de convolution (CONV) suivante, à la carte d'activation (FM) issue d'une couche de convolution (CONV) correspondante du chemin de contraction (CP) au travers d'un saut de connexion (SC) entre le chemin de contraction (CP) et le chemin d'expansion (EP).

4. Procédé selon l'une des revendications précédentes, **caractérisé en ce qu'**il comprend, à l'issue de l'étape de segmentation (E1) et au préalable de l'étape de comparaison (E3), une étape de confirmation et de correction (E2) des étiquettes attribuées par le classifieur (CNN) aux voxels de la représentation tridimensionnelle (1).

5. Procédé selon l'une des revendications précédentes, dans lequel l'étape de comparaison (E3) comporte :
a. une première sous-étape de comparaison (E31) de la valeur de chaque voxel d'une pluralité de voxels de la représentation tridimensionnelle (1), dont les étiquettes attribuées sur la cartographie tridimensionnelle (2) sont celles du vaisseau sanguin (AA), à une première valeur seuil prédéterminée, une première étiquette associée à un stent étant attribuée à chaque voxel dont la valeur dépasse ladite première valeur seuil prédéterminée ;
b. une deuxième sous-étape de comparaison (E32) de la valeur de chaque voxel d'une pluralité de voxels de la représentation tridimensionnelle, dont les étiquettes attribuées sur la cartographie tridimensionnelle sont celles du vaisseau sanguin, à une deuxième valeur seuil prédéterminée inférieure à la première valeur seuil, une deuxième étiquette associée à une calcification étant attribuée à chaque voxel dont la valeur dépasse ladite deuxième valeur seuil prédéterminée.

6. Procédé selon l'une des revendications précédentes, dans laquelle l'étape de comparaison (E2) est mise en œuvre pour une pluralité de voxels dont les étiquettes attribuées sur la cartographie tridimensionnelle (2) sont celles de la lumière (L) du vaisseau sanguin (AA) et sont situées au niveau d'une frontière de délimitation de la cartographie tridimensionnelle (2) entre les étiquettes (2L) de la lumière (L) et les étiquettes (2T) des tuniques (T) du vaisseau sanguin.

7. Procédé selon l'une des revendications précédentes, dans lequel l'étape de détermination (E4) de l'évolution d'un indicateur géométrique (Di, Dj) du vaisseau sanguin (AA) est une étape de détermination de l'évolution du diamètre du vaisseau sanguin et comporte une étape (E41) d'estimation d'un graphe (3) parcourant l'intégralité du vaisseau sanguin et dont chaque point (3i) est le barycentre des voxels situés dans une section (C3i) de la représentation tridimensionnelle (1) localement orthogonale au graphe et dont les étiquettes sont celles du vaisseau sanguin (AA) ; dans lequel un diamètre local du vaisseau sanguin est déterminé en fonction de chacun des points du graphe.

8. Procédé selon la revendication précédente, dans lequel l'étape de détermination (E4) comporte en outre une étape (E42) d'estimation d'un nuage de points (4), chaque point (4i) du nuage de points étant le point localement le plus éloigné d'une frontière des voxels de la représentation tridimensionnelle (1) du vaisseau sanguin (AA) et dont les étiquettes sont celles du vaisseau sanguin et une étape (E43) de correction des points (3i) du graphe (3) à l'aide du nuage de points.

## Patentansprüche

1. Computergestütztes Verfahren zur Unterstützung der Diagnose einer Herz-Kreislauf-Erkrankung (A) eines Blutgefäßes (AA), umfassend die folgenden Schritte:
a. (E0) Bereitstellung einer dreidimensionalen Darstellung (1) eines Blutgefäßes (AA) eines Patienten, die mit einer medizinischen Bildgebungsvorrichtung erhalten wurde;
b. (E1) Segmentierung der dreidimensionalen Darstellung unter Verwendung eines Klassifikators (CNN), um eine segmentierte dreidimensionale Karte (2) der dreidimensionalen Darstellung zu erhalten, wobei der Klassifikator angeordnet ist, um zu schätzen, ob jedes Voxel der dreidimensionalen Darstellung zu dem Blutgefäß gehört und dieses Voxel in Abhängigkeit von dieser Schätzung zu kennzeichnen, wobei die segmentierte dreidimensionale Karte aus der Gesamtheit der vom Klassifikator den Voxeln der dreidimensionalen Darstellung zugewiesenen Bezeichnungen gebildet wird;
c. (E3) Vergleich des Wertes jedes Voxels einer Vielzahl von Voxeln der dreidimensionalen Darstellung, deren zugewiesene Bezeichnungen auf der dreidimensionalen Karte die des Blutgefäßes sind, mit einem vorbestimmten Schwellenwert, wobei jedem Voxel, dessen Wert den vorbestimmten Schwellenwert überschreitet, eine andere Bezeichnung als die des Blutgefäßes zugewiesen wird;
d. (E4) Bestimmung der Entwicklung eines geometrischen Indikators (Di, Dj) des Blutgefäßes entlang dieses Blutgefäßes mittels der Voxel der dreidimensionalen Darstellung, deren zugewiesene Bezeichnungen auf der dreidimensionalen Karte die des Blutgefäßes sind;
wobei der Klassifikator (CNN) angeordnet ist, um für jedes Voxel der dreidimensionalen Darstellung (1) zu schätzen, ob:
i. dieses Voxel außerhalb des Blutgefäßes (AA) liegt, wobei der Klassifikator diesem Voxel in diesem Fall eine erste Bezeichnung zuweist,
ii. dieses Voxel zum Lumen (L) des Blutgefäßes gehört, wobei der Klassifikator diesem Voxel in diesem Fall eine zweite Bezeichnung zuweist,
iii. dieses Voxel zu einer Tunica (T) des Blutgefäßes gehört, wobei der Klassifikator diesem Voxel in diesem Fall eine dritte Bezeichnung zuweist;
wobei der Segmentierungsschritt (E1) durch einen Klassifikator (CNN) implementiert wird, der einen maschinellen Lernalgorithmus implementiert,
und wobei der Segmentierungsschritt (E1) die Segmentierung (E12) mittels des Klassifikators (CNN) von drei axialen, sagittalen und koronalen Schnitten (IS) der dreidimensionalen Darstellung (1) umfasst, um drei segmentierte zweidimensionale Karten (CB) zu erhalten, und einen Kombinationsschritt (E13) der zweidimensionalen Karten, um die dreidimensionale Karte (2) zu erhalten.

2. Verfahren nach dem vorstehenden Anspruch, wobei der Klassifikator (CNN) ein faltendes neuronales Netzwerk ist, umfassend einen Kontraktionspfad (CP) und einen Expansionspfad (EP), wobei der Kontraktionspfad eine Vielzahl von Faltungsschichten (CONV) umfasst, die jeweils einer Korrekturschicht (RELU) zum Implementieren einer Aktivierungsfunktion und Downsampling-Schichten (POOL) zugeordnet sind, wobei auf jede Downsampling-Schicht mindestens eine Faltungsschicht folgt, wobei der Expansionspfad eine Vielzahl von Faltungsschichten (CONV) und Upsampling-Schichten (UPSAMP) umfasst, wobei auf jede Upsampling-Schicht eine Faltungsschicht folgt.

3. Verfahren nach dem vorstehenden Anspruch, wobei die Abgabe jeder Upsampling-Schicht (UPSAMP) vor dem Eintritt in die nachfolgende Faltungsschicht (CONV) mit der Aktivierungskarte (FM) einer entsprechenden Faltungsschicht (CONV) des Kontraktionspfads (CP) über einen Verbindungssprung (SC) zwischen dem Kontraktionspfad (CP) und dem Expansionspfad (EP) verkettet wird.

4. Verfahren nach einem der vorstehenden Ansprüche,
**dadurch gekennzeichnet, dass** es nach dem Segmentierungsschritt (E1) und vor dem Vergleichsschritt (E3) einen Bestätigungs- und Korrekturschritt (E2) der vom Klassifikator (CNN) den Voxeln der dreidimensionalen Darstellung (1) zugewiesenen Bezeichnungen umfasst.

5. Verfahren nach einem der vorstehenden Ansprüche, wobei der Vergleichsschritt (E3) umfasst:
a. einen ersten Vergleichsteilschritt (E31) des Wertes jedes Voxels einer Vielzahl von Voxeln der dreidimensionalen Darstellung (1), deren zugewiesene Bezeichnungen auf der dreidimensionalen Karte (2) die des Blutgefäßes (AA) sind, mit einem ersten vorbestimmten Schwellenwert, wobei jedem Voxel, dessen Wert den ersten vorbestimmten Schwellenwert überschreitet, eine erste Bezeichnung zugewiesen wird, die einem Stent zugeordnet ist;
b. einen zweiten Vergleichsteilschritt (E32) des Wertes jedes Voxels einer Vielzahl von Voxeln der dreidimensionalen Darstellung, deren zugewiesene Bezeichnungen auf der dreidimensionalen Karte die des Blutgefäßes sind, mit einem zweiten vorbestimmten Schwellenwert, der niedriger als der erste Schwellenwert ist, wobei jedem Voxel, dessen Wert den zweiten vorbestimmten Schwellenwert überschreitet, eine zweite Bezeichnung zugewiesen wird, die einer Verkalkung zugeordnet ist.

6. Verfahren nach einem der vorstehenden Ansprüche, wobei der Vergleichsschritt (E2) für eine Vielzahl von Voxeln implementiert wird, deren zugewiesene Bezeichnungen auf der dreidimensionalen Karte (2) die des Lumens (L) des Blutgefäßes (AA) sind und die sich an einer Grenze der dreidimensionalen Karte (2) zwischen den Bezeichnungen (2L) des Lumens (L) und den Bezeichnungen (2T) der Tunicae (T) des Blutgefäßes befinden.

7. Verfahren nach einem der vorstehenden Ansprüche, wobei der Bestimmungsschritt (E4) der Entwicklung eines geometrischen Indikators (Di, Dj) des Blutgefäßes (AA) ein Bestimmungsschritt der Entwicklung des Durchmessers des Blutgefäßes ist und einen Schätzungsschritt (E41) eines Graphen (3) umfasst, der das gesamte Blutgefäß abdeckt und jeder Punkt (3i) davon das Baryzentrum der Voxel ist, die sich in einem Schnitt (C3i) der dreidimensionalen Darstellung (1) befinden, der lokal orthogonal zum Graphen ist und dessen Bezeichnungen die des Blutgefäßes (AA) sind; wobei ein lokaler Durchmesser des Blutgefäßes in Abhängigkeit von jedem Punkt des Graphen bestimmt wird.

8. Verfahren nach dem vorstehenden Anspruch, wobei der Bestimmungsschritt (E4) ferner einen Schätzungsschritt (E42) einer Punktwolke (4) umfasst, wobei jeder Punkt (4i) der Punktwolke der lokal am weitesten von einer Grenze der Voxel der dreidimensionalen Darstellung (1) des Blutgefäßes (AA) entfernte Punkt ist und deren Bezeichnungen die des Blutgefäßes sind, und einen Korrekturschritt (E43) der Punkte (3i) des Graphen (3) unter Verwendung der Punktwolke.

## Claims

1. Computer-implemented method for aiding in the diagnosis of a cardiovascular disease (A) of a blood vessel (AA), the method comprising the following steps:
a. (E0) Providing a three-dimensional representation (1) of a blood vessel (AA) of a patient, obtained by a medical imaging device;
b. (E1) Segmenting, by means of a classifier (CNN), said three-dimensional representation to obtain a segmented three-dimensional map (2) of said three-dimensional representation, the classifier being arranged to estimate whether each voxel of the three-dimensional representation belongs to said blood vessel and to label this voxel according to this estimate, said segmented three-dimensional map being formed by the set of labels assigned by the classifier to the voxels of the three-dimensional representation;
c. (E3) Comparing the value of each voxel of a plurality of voxels of the three-dimensional representation, the labels of which assigned on the three-dimensional map are those of the blood vessel, with a predetermined threshold value, a label different from those of the blood vessel being assigned to each voxel, the value of which exceeds said predetermined threshold value;
d. (E4) Determining the evolution of a geometric indicator (Di, Dj) of the blood vessel along this blood vessel by means of the voxels of the three-dimensional representation, the labels of which assigned on the three-dimensional map are those of the blood vessel;
wherein the classifier (CNN) is arranged to estimate, for each voxel of the three-dimensional representation (1), whether:
i. this voxel is outside the blood vessel (AA), in which case the classifier assigns a first label to this voxel,
ii. this voxel belongs to the lumen (L) of the blood vessel, in which case the classifier assigns a second label to this voxel,
iii. this voxel belongs to a tunica (T) of the blood vessel, in which case the classifier assigns a third label to this voxel;
wherein the segmentation step (E1) is implemented by a classifier (CNN) implementing a machine learning algorithm
and wherein the segmentation step (E1) comprises segmenting (E12), by means of the classifier (CNN), three axial, sagittal and coronal sections (IS) of said three-dimensional representation (1) to obtain three segmented two-dimensional maps (CB) and a step (E13) of combining the two-dimensional maps to obtain said three-dimensional map (2).

2. Method according to the preceding claim, wherein the classifier (CNN) is a convolutional neural network, comprising a contraction path (CP) and an expansion path (EP), wherein the contraction path comprises a plurality of convolutional layers (CONV) each associated with a correction layer (RELU) arranged to implement an activation function and downsampling layers (POOL), each downsampling layer being followed by at least one convolutional layer, wherein the expansion path comprises a plurality of convolutional layers (CONV) and upsampling layers (UPSAMP), each upsampling layer being followed by a convolutional layer.

3. Method according to the preceding claim, wherein the output of each upsampling layer (UPSAMP) is concatenated, before entering the next convolutional layer (CONV), to the activation map (FM) from a corresponding convolutional layer (CONV) of the contraction path (CP) via a skip connection (SC) between the contraction path (CP) and the expansion path (EP).

4. Method according to any of the preceding claims, **characterized in that** it comprises, at the end of the segmentation step (E1) and prior to the comparison step (E3), a step (E2) of confirming and correcting the labels assigned by the classifier (CNN) to the voxels of the three-dimensional representation (1).

5. Method according to any of the preceding claims, wherein the comparison step (E3) comprises:
a. a first sub-step (E31) of comparing the value of each voxel of a plurality of voxels of the three-dimensional representation (1), the labels of which assigned on the three-dimensional map (2) are those of the blood vessel (AA), with a first predetermined threshold value, a first label associated with a stent being assigned to each voxel, the value of which exceeds said first predetermined threshold value;
b. a second sub-step (E32) of comparing the value of each voxel of a plurality of voxels of the three-dimensional representation, the labels of which assigned on the three-dimensional map are those of the blood vessel, with a second predetermined threshold value lower than the first threshold value, a second label associated with calcification being assigned to each voxel, the value of which exceeds said second predetermined threshold value.

6. Method according to any of the preceding claims, wherein the comparison step (E2) is carried out for a plurality of voxels, the labels of which assigned on the three-dimensional map (2) are those of the lumen (L) of the blood vessel (AA) and are located at a delimiting boundary of the three-dimensional map (2) between the labels (2L) of the lumen (L) and the labels (2T) of the tunicas (T) of the blood vessel.

7. Method according to any of the preceding claims, wherein the step (E4) of determining the evolution of a geometric indicator (Di, Dj) of the blood vessel (AA) is a step of determining the evolution of the diameter of the blood vessel and comprises a step (E41) of estimating a graph (3) traversing the entire blood vessel and each point (3i) of which is the barycenter of the voxels located in a section (C3i) of the three-dimensional representation (1) locally orthogonal to the graph and the labels of which are those of the blood vessel (AA); wherein a local diameter of the blood vessel is determined on the basis of each of the points on the graph.

8. Method according to the preceding claim, wherein the determination step (E4) further comprises a step (E42) of estimating a point cloud (4), each point (4i) of the point cloud being the point which is locally furthest from a voxel boundary of the three-dimensional representation (1) of the blood vessel (AA) and the labels of which are those of the blood vessel, and a step (E43) of correcting the points (3i) on the graph (3) using the point cloud.
